# EUROPEAN PATENT APPLICATION

(11) **EP 0 526 888 A2**
(43) Date of publication of application: **10.02.1993**
(21) Application number: 92113367.4
(22) Date of filing: 05.08.1992
(51) Int. Cl.: C12P 21/08, G01N 33/574, A61K 39/395, A61K 47/42

(54) **Monoclonal antibody reactive with human pancreas cancer tissue**

(30) Priority: 06.08.1991 JP 196824/91; 21.10.1991 JP 272620/91
(71) Applicant: TORAY INDUSTRIES, INC., Tokyo 103 (JP)
(72) Inventor: Sato, Nahoko, Kamakura-shi, Kanagawa 248 (JP); Uenishi, Noriaki, Kamakura-shi, Kanagawa 248 (JP); Yamazaki, Shojiro, Kamakura-shi, Kanagawa 248 (JP)
(74) Representative: Kador & Partner

(57) **Abstract**

Monoclonal Antibodies specific to human pancreas cancer are disclosed. The monoclonal antibodies of the present invention react with human pancreas cancer tissue with positive rates of not less than 75% determined by immunohistostaining. The monoclonal antibodies of the present invention are useful as components of diagnostics for human pancreas cancer and pharmaceuticals for treating human pancreas cancer.

## Description

### BACKGROUND OF THE INVENTION

### I. Field of the Invention

This invention relates to a monoclonal antibody which strongly reacts with human pancreas cancer tissue and is useful for diagnosis and therapy of human pancreas cancer.

### II. Description of the Related Art

Therapeutic methods such as chemotherapy, radiotherapy, surgical therapy and immunotherapy are now widely performed for the treatment of cancers which are the most common causes of death in this country have been advanced year by year. As for the chemotherapy, various anti-cancer agents have been provided. Recently, studies for treating cancers with tumor-specific monoclonal antibodies are intensively made and monoclonal antibodies as pharmaceuticals are now being developed. The objective organs to be treated include wide variety of organs, e.g., hematogenic cells, lung, liver, digestive tract, ovary and prostate. However, no effective chemotherapeutic agent for pancreas cancer has not yet been provided, so that a highly effective agent against this cancer is strongly required. Further, since most of chemotherapeutic agents cause side effects such as reduction of the number of leukocytes or platelets, a therapeutic method employing a tumor-specific monoclonal antibody is preferred.

The known therapeutic methods for cancers employing a monoclonal antibody include the methods in which an antibody alone is administered so as to accumulate macrophages or lymphocytes having receptors for the Fc region of the antibody in the vicinity of the tumor tissue, thereby attacking the cancer, and the methods so called target therapy in which the cancer is treated with an immunotoxin conjugate containing a monoclonal antibody to which a toxic substance such as a toxin protein, radioisotope (RI), anti-cancer agent or the like is bound.

The known diagnostic methods for cancers employing a monoclonal antibody include in vitro diagnostics utilizing a monoclonal antibody which specifically reacts with tumor markers in blood, pathological diagnostics utilizing a monoclonal antibody which specifically reacts with tumor cells in pathological tissue specimen, and in vivo diagnostics (RI-imaging) which gives the result in accumulation of monoclonal antibody into tumor organ when the monoclonal antibody labeled with radioisotope (RI) is administered. Especially for diagnosis of pancreas cancer, it is said that in vitro diagnostic is not useful to identify the cancer but that pathological diagnostics and in vivo diagnostic (imaging) are more useful than in vitro diagnostic.

However, up to now, few monoclonal antibodies which specifically react with pancreas cancer and which are effective for the treatment of pancreas cancer are known. Only several monoclonal antibodies such as anti-CA19-9 antibody and anti-Dupan-2 antibody are known to react with pancreas cancer. Although these antibodies are useful as in vitro diagnostics, it is doubtful whether they are valuable for pathological diagnostics, in vivo diagnostics, and the therapy of pancreas cancer (Herlyn, M. et al., J. Nat. Cancer Inst., 82, 1883-1889 (1990)) because they recognize sugar of cancer associated antigens. Thus, a monoclonal antibody which strongly reacts with pancreas cancer tissue and which is effective for the diagnosis and therapy of pancreas cancer is desired to be developed.

### SUMMARY OF THE INVENTION

The object of the present invention is to discover a novel monoclonal antibody which specifically reacts with human pancreas cancer tissue and is effective for detecting and treating human pancreas cancer.

The above-mentioned object is attained by the present invention which follows. That is, the present invention provides a monoclonal antibody which reacts with human pancreas cancer tissue with a positive rate of not less than 75% determined by immunohistostaining, and furthermore, provides diagnostic agent to detect human pancreas cancer which is used for in vitro diagnostics including pathological method, and in vivo diagnostic including RI-imaging.

Since the monoclonal antibody of the present invention specifically and strongly reacts with human pancreas cancer tissue, the monoclonal antibody of the present invention is suited for the therapy of pancreas cancer.

That is, by virtue of the monoclonal antibody of the present invention, a method for diagnosing pancreas cancer comprising immunohistostaining the pancreas of an individual with the monoclonal antibody of the present invention is provided. Also provided is a method for treating pancreas cancer comprising administering the monoclonal antibody of the present invention to a patient suffering from pancreas cancer. A method for treating pancreas cancer comprising administering an immunotoxin conjugate having the monoclonal antibody of the present invention and a toxin to a patient suffering from pancreas cancer is provided. Further, a diagnostic for pancreas cancer comprising the monoclonal antibody of the present invention and a label is provided. Still further, a pharmaceutical for treating pancreas cancer comprising the monoclonal antibody of the present invention as an effective ingredient is provided. Still further, a pharmaceutical for treating pancreas cancer comprising an immunotoxin conjugate having the monoclonal antibody of the present invention and a toxin as an effective ingredient is provided.

### DETAILED DESCRIPTION OF THE PREFERRED EMBODIMENTS

The monoclonal antibody of the present invention reacts with human pancreas cancer tissue with a positive rate of not less than 75% determined by immunohistostaining, and is preferably a monoclonal antibody which recognizes a polypeptide portion of a surface molecule of the tumor cells. The monoclonal antibody of the present invention is also preferably one which is obtained by immunizing an animal with a human hepatocellularcarcinoma cell line. The term "positive rate" herein means the percentage of pancreas cancer patients who have pancreas cancer tissue which reacts with the monoclonal antibody in total pancreas cancer patients, the reaction being determined by an ordinary immunohistostaining. The monoclonal antibody of the present invention exhibits the positive rate of not less than 75%, preferably not less than 80%.

In the present invention, SF25 (ATCC HB 9599), XF8 (ATCC HB 9686) and AF20 (ATCC HB 9687) which are obtained by using human hepatocellularcarcinoma cells as immunogens (Takahashi H. et al., Cancer Res., 48, 6573-6579 (1988), Takahashi H. et al., Hepatology, 9, 625-634 (1989)) are preferably employed. These monoclonal antibodies are produced by monoclonal antibody-producing hybridomas obtained by the known cell fusion method. More particularly, human hepatocellularcarcinoma cell line FOCUS (Wilson B., et al., Proc. Natl. Acad. Sci. USA 85, 314 0-3144 (1988)) is cultured in a flask and the cells are then harvested. The cells are intraperitoneally administered to a mouse and then the spleen of the mouse is taken. The spleen cells are fused with myeloma cells and the produced hybridomas having antibody-producing ability and proliferation ability are selected. Thereafter, the hybridomas are cloned by the limiting dilution method or the like so as to obtain the desired monoclonal antibody-producing hybridoma.

It is known that the little or no antigen which these three antibodies recognize exist in the normal lung, liver, intestine, spleen, kidney and the like, but specifically exist in cancerous tissues, as shown by the results of the pathological histostaining of human organs by the antibodies SF25, XF8 and AF20 (Takahashi, H. et al., Cancer Res., 48, 6573-6579 (1988), Takahashi H. et al., Hepatology, 9, 625-634 (1989)), that the antibodies strongly react with hepatocellularcarcinoma, cancers of digestive tract and lung cancer as shown by the reactivity with cancer cell lines, and that these findings were also confirmed by the immunostaining of frozen human cancer tissue sections. However, the fact that these antibodies have strong reactivity with human pancreas cancer tissue is not known and was discovered by the present inventors. Further, the positive rates of reaction with each cell are high and the positive rates with pancreas cancer tissue are also very high. Further, the three monoclonal antibodies recognize peptide portions of surface molecules of the cells (Takahashi, H. et al., Cancer Res., 48, 6573-6579 (1988), Takahashi H. et al., Hepatology, 9, 625-634 (1989)), so that the antigen is hardly detached from the cell surfaces.

The monoclonal antibody of the present invention may be derived from any species such as mouse and human. Further, the monoclonal antibody may be a chimeric antibody prepared by the genetic recombination technique, in which the amino acid sequence of the constant region is replaced with the other amino acid sequence.

The diagnostic methods for the cancer using the monoclonal antibody include a method of in vitro diagnosis to detect tumor markers in the serum by immuno-chemical determination such as RIA, EIA and so on, and a method of pathological diagnosis by immuno-histochemical staining on sample slides of thin tissue section. In recently, there is also a method which radio-isotope (RI)-labeled monoclonal antibody is injected into the patient and is accumulated into the tumor in a deep part of body, so-called in vivo diagnostics (RI-imaging). The RI used for the imaging are ¹¹¹In, ^{99m}Tc and etc, and the labelling method to obtain RI-antibody are a chelating method that chelate compound such as DTPA binds to antibody and then forms chelate-bonding with RI, and recently Schwartz' method [S.J. Mather and D.Ellison, J. Nucl. Med., 31 692-697, (1990)] which RI reacts with antibody after exposed chelation site in the antibody molecule. Moreover, as for an antibody, whole IgG, Fab' and etc are properly chosen in consideration of their metabolic fate in the body.

The therapeutic methods for the cancer using the monoclonal antibody include a method in which the antibody alone is administered so as to accumulate the macrophages or lymphocytes having the receptor to the Fc region of the antibody in the vicinity of the tumor, thereby attacking the cancer, the method being known as immunotherapy. In this method, chimeric antibodies having the Fc region of human IgG₁ are preferably employed.

The therapeutic methods also include a method known as target therapy in which an immunotoxic conjugate having a toxic substance such as toxin protein, radioisotope (RI) or an anti-cancer agent is conjugated to the monoclonal antibody is employed. In this case, any antibody including mouse antibodies and chimeric antibodies may be employed as long as it contains the binding region to the tumor cells. For example, IgG, F(ab')₂, Fab' and Fab may be employed. The toxin proteins which may be used as the toxic substance include A chains of plant toxins (ribosome-inactivating proteins type II) such as ricin and abrin, plant toxins (ribosome-inactivating proteins type I) such as luffin, momordin, and PAP-S, and A chains of bacterial toxin proteins such as Pseudomonas toxin and diphtheria toxin. The toxins also include human-derived cytotoxic proteins such as ribonuclease from human pancreas, which kills the cell when it is taken into the cells by binding to monoclonal antibody. These toxins irreversibly stop the protein synthesis, so that they exhibit strong toxicity against cells.

For the preparation of an immunotoxin conjugate by binding the toxin protein and the antibody, a cross-linking agent [Protein, Nucleic Acid, Enzyme, extra edition No. 31, 335-343 (1987)] is employed. After the immunotoxin conjugate is bound to the tumor cells, the immunotoxin conjugate is rapidly taken into the cells and migrates to the site where the toxin exerts its activity, thereby killing the tumor cells. Thus, the monoclonal antibody preferably has a property to be rapidly taken into the cells after bound to the cells.

Radioisotopes (RI) and anti-cancer agents may also be used as the toxic substance. In these cases, the antibody need not be taken into the cells, but the effects are well exhibited if the antibody reaches to the vicinity of the cells. However, the amount of the anti-cancer agent reaching to the cells must be sufficient. Thus, the more the number of molecules of the anti-cancer agent to be bound to the antibody, the better. Thus, several tens to several hundreds of the molecules of the anti-cancer agent may be bound to the antibody. To accomplish this, a high polymer to which a number of molecules of the anti-cancer agent are attached may be bound to the antibody.

The invention will now be described more concretely by way of examples thereof. It should be noted, however, the examples are presented for the illustration purpose only and should not be interpreted in any restrictive way.

### Example 1

### Reactivity of the monoclonal antibodies of the present invention with human pancreas cancer tissue

The monoclonal antibodies used in this example are monoclonal antibodies SF25, XF8 and AF20 corresponding to the surface antigen of human hepatocellularcarcinoma cell line FOCUS, which were prepared according to the method by Takahashi et al (see H. Takahashi et al., Hepatology, 9, 625 (1989)).

The preparation of the frozen tissue section specimens and the immunohistostaining were performed as follows.

After cutting out fresh tissue of human pancreas cancer, the tissue is immediately frozen and stored. Frozen sections of the thus obtained tissue are prepared and fixed with acetone at -20^{o}C for 5 minutes, followed by washing with PBS(-). The sections are then reacted with 2% horse serum at room temperature for 20 minutes. The sections are reacted with 0.5 µg/ml of a primary antibody, that is, SF25, XF8 or AF20 at 4^{o}C overnight, and then washed with PBS(-). Thereafter, the sections are reacted with a 400-fold diluted biotin-bound secondary antibody at room temperature for 3 hours and then washed with PBS(-). The sections are then treated with methanol containing 0.3% H₂O₂ at room temperature for 20 minutes and washed with PBS(-). The sections are then reacted with 200-fold diluted ABC (avidin-biotin complex) reagent for one and half hour and then washed with PBS(-), followed by the treatment with a substrate solution containing DAB (3,3'-diaminobenzidine) which is the substrate of the enzyme and hydrogen peroxide, so as to stain the sections. The results are shown in Table 1.

As shown in Table 1, all of the three antibodies, SF25, XF8 and AF20, exhibited high reactivity as high as about 80% or more with the human pancreas cancer tissue. Further, they well react with the tissue after treated by RI irradiation. Since the reactivity of the antigen is not lost by the RI irradiation, it is expected that these antibodies may be widely applied for the therapy. In the fractions shown in Table 1, the denominators indicate the total number of the patients whose pancreas tissues were stained, and the numerators indicate the number of patients having the tissues stained. The values in parentheses indicate the positive rates.

**Table 1**

| Reactivity of the Monoclonal Antibodies with Human Pancreas Cancer Tissue | | | |
|---|---|---|---|
| Position of Tumor | Monoclonal Antibodies | | |
| | SF25 | XF8 | AF20 |
| Pancreas Cancer Tissue | 17/19 (89) | 15/19 (79) | 17/19 (89) |
| Primary Lesion | 3/3 | 1/3 | 3/3 |
| RI-irradiated Cancer Tissue | 8/9 | 7/9 | 7/9 |
| Pancreas Cancer Metastasized to Other Organs | 6/7 | 7/7 | 7/7 |

### Example 2

### Anticellular Effects of Antibody-Toxin Conjugate Against Human Pancreas Cancer Cell Lines

Using three pancreas cancer cell lines, BxPC-3, PANC-1 and MIAPaCa, the anticellular effects of AF20 antibody-toxin conjugate were examined by the following method:
Cultured cells of each of the cell lines were placed in the wells of a 24-well plate in a cell population of 5 x 10³ cells/ml/well, and the cells were cultured at 37^{o}C under atmosphere of 5%CO₂. On the next day, serially diluted antibody-toxin conjugate (AF20-abrin A chain conjugate (hereinafter designated as "AF20-AA") or AF20-ricin A chain conjugate (hereinafter designated as "AF20-RA")) was added to the wells in an amount of 0.1 ml/well. One day after the addition of the immunotoxins, each well was washed twice with the fresh culture medium and another fresh culture medium was placed in each well. The cells were cultured for another 5 days and then the number of cells were counted with a Coulter counter. As a control, PBS was added to a well in place of the immunotoxin and the number of the cells in the well was also counted in the same manner. From the numbers of the cells in the wells, IC₅₀ values were determined. The IC₅₀ value means the concentration of the immunotoxin at which the number of cells is reduced to 50% of the number of the cells in the control well.

AF20-AA and AF20-RA were prepared by the following methods:

### Preparation of AF20-AA

### A. Abrin A Chain

Pulverized Abrus precatorius seeds were defatted by the extraction with petroleum ether, and then immediately extracted with water. In the extraction, the pH was adjusted to 4.5 with acetic acid. After centrifugation, the supernatant was recovered and was successively subjected to precipitation with ammonium sulfate (40 - 70%), to affinity chromatography by acid-treated Sepharose 4B, and to that by DEAE cellulose, so as to purify abrin. The thus obtained abrin was reduced with Tris buffer (pH 7.7) containing 5% mercaptoethanol at room temperature for 3 hours to overnight. Thereafter, the pH was adjusted to 8.5 and the resultant was passed through DEAE cellulose column, followed by extensive washing. Thereafter, elution was performed with buffer (pH 8.5) containing 0 - 0.2M NaCl, and the eluted solution was then passed through asialofetuin column, followed by gel permeation chromatography, thereby obtaining purified abrin A chain. The analysis by SDS-PAGE employing silver staining revealed that no abrin B chain was contaminated in the purified abrin A chain.

### B. Preparation of Immunotoxin Conjugate

Conjugate of the abrin A chain and AF20 antibody, that is, AF20-AA was prepared by using SPDP (N-succinimidyl-3-(2-pyridyldithio)propionate) as a cross-linking agent. More particularly, antibody solution in PBS was mixed with SPDP solution in dimethylformamide with a mixing ratio of 4:1 in terms of the molar ratio of SPDP to the antibody, and the resulting mixture was left to stand at room temperature for 30 minutes to allow the reaction, followed by gel permeation chromatography. Thereafter, three-fold molar of abrin A chain was added into the antibody, and the resultant was left to stand at room temperature overnight so as to allow the reaction, thereby preparing a conjugate. The obtained conjugate was purified by "Blue Sepharose CL-6B" column and then by "Ultrogel AcA44" column as in Example 1 to finally obtain purified immunotoxin conjugate. The thus obtained immunotoxin conjugate contained one or two abrin A chains per one molecule of the antibody. The analysis by SDS-PAGE employing silver staining revealed that the immunotoxin conjugate did not contain free ricin A chain.

### Preparation of AF20-RA

### A. Ricin A Chain

Castor-oil plant bean lectin RCA-60 purchased from Seikagaku Kogyo Co., Ltd. was fixed to a lactose-agarose column and the column was treated with PBS containing 5% mercaptoethanol (ME-PBS) overnight. Then the ricin A chain alone was eluted by ME-PBS. The eluted solution was passed through an asialofetuin column to obtain purified ricin A chain. The analysis by SDS-PAGE employing silver staining revealed that no ricin B chain was contaminated in the purified ricin A chain.

### B. Preparation of Immunotoxin Conjugates

Conjugate of the ricin A chain and AF20 antibody was prepared by using SPDP as a cross-linking agent. More particularly, antibody solution in PBS was mixed with SPDP solution in dimethylformamide with a mixing ratio of 4:1 in terms of the molar ratio of SPDP to the antibody, and the resulting mixture was left to stand at room temperature for 30 minutes to allow the reaction, followed by gel permeation chromatography. Thereafter, three-fold molar of ricin A chain was added into the antibody under condition of neutral pH, and the resultant was left to stand at room temperature overnight so as to allow the reaction, thereby preparing a conjugate. The obtained conjugate was purified by "Blue Sepharose CL-6B" column (commercially available from Pharmacia) and "Ultrogel AcA44" column (commercially available from IBF Biotechnics) to finally obtain purified immunotoxin conjugate. Each of the thus obtained immunotoxin conjugates contained one or two ricin A chains per one molecule of the antibody. The analysis by SDS-PAGE employing silver staining revealed that neither of the immunotoxin conjugates contained free ricin A chain.

The determined IC₅₀ values are shown in Table 2 below.

**Table 2**

| Anticellular Effects of Antibody-Toxin Conjugates Against Pancreas Cancer Cells | | | |
|---|---|---|---|
| Cell Line | Origin | IC₅₀ (ng/ml) | |
| | | AF20-AA | AF20-RA |
| BxPC-3 | Pancreas Cancer | 6.4 | 1.7 |
| PANC-1 | Pancreas Cancer | 10.7 | 2.0 |
| MIAPaCa | Pancreas Cancer | 20.4 | 2.3 |

As can be seen from Table 2, the immunotoxin conjugates AF20-AA and AF20-RA exhibited anticellular effects against pancreas cancer cells.

## Claims

1. A monoclonal antibody which reacts with human pancreas cancer tissue with a positive rate of not less than 75% determined by immunohistostaining.

2. The monoclonal antibody of claim 1, which recognizes a polypeptide portion of a surface molecule of tumor cells.

3. The monoclonal antibody of claim 1 or 2, which is prepared by immunizing an animal with a human hepatocellularcarcinoma cell line.

4. The monoclonal antibody of any one of claims 1 - 3, which is SF25, XF8 or AF20.

5. An agent for the diagnosis of human pancreas cancer comprising the monoclonal antibody of any one of claims 1 - 4 as a main component.

6. A pharmaceutical for treating human pancreas cancer comprising said monoclonal antibody of claim 1 as an effective ingredient.

7. A pharmaceutical for treating human pancreas cancer comprising as an effective ingredient an immunotoxin conjugate having said monoclonal antibody of claim 1 and a toxin A chain attached to said monoclonal antibody.

8. The pharmaceutical of claim 7, wherein said toxin A chain is ricin A chain or abrin A chain.
